(19) Europäisches Patentamt
European Patent Office
Office européen des brevets

(11) Publication number : **0 475 664 B1**

(12) # EUROPEAN PATENT SPECIFICATION

(45) Date of publication of patent specification : **10.11.93 Bulletin 93/45**

(51) Int. Cl.$^5$ : **A61K 9/02,** A61K 47/10, A61K 47/24, A61K 47/34, A61K 7/48, C10M 169/04

(21) Application number : **91308082.6**

(22) Date of filing : **04.09.91**

(54) **Lubricant composition and use thereof.**

(30) Priority : **10.09.90 FR 9011172**

(43) Date of publication of application : **18.03.92 Bulletin 92/12**

(45) Publication of the grant of the patent : **10.11.93 Bulletin 93/45**

(84) Designated Contracting States : **DE ES FR GB IT NL SE**

(56) References cited :
**EP-A- 0 259 734**
**GB-A- 1 043 513**

(73) Proprietor : **Dow Corning France S.A.**
**Route des Cretes BP 43 Sophia Antipolis Les Bouillides**
**F-06561 Valbonne (FR)**

(72) Inventor : **Aguadisch, Louis Michel Jacques**
**720 A. Chemin de Villebruc**
**F-06560 Valbonne (FR)**
Inventor : **Etienne, Alain**
**24 Route de Pegomas**
**F-06130 Grasse (FR)**

(74) Representative : **Vandamme, Luc Johan Roger**
**Dow Corning Limited Cardiff Road**
**Barry South Glamorgan CF6 7YL Wales (GB)**

EP 0 475 664 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to lubricant compositions and is particularly concerned with lubricant compositions having spermicidal or virucidal properties.

It is known that certain polyethyleneoxy alkylphenols have spermicidal properties and are thus useful for incorporation into lubricants for condoms. G.B. Patent Specification 1 043 513 describes such compositions wherein the polyethyleneoxy alkylphenol is dispersed in a water insoluble polysiloxane polyoxyalkylene copolymer. Although silicone liquids, in particular trimethylsiloxy end-blocked polydimethylsiloxanes, are known to lubricate elastomeric and plastic materials they are not compatible with certain compounds having spermicidal or virucidal activity. Combining silicone liquids and polyethyleneoxy alkylphenols have tended to lead to dispersions which are unstable and which have a milky, rather than a more desirable clear appearance.

We have now found that organosilicon compounds having silicon-bonded hydroxyl groups can be used as carriers for certain polyoxyethylene alkylphenols to provide spermicidal and virucidal compositions which are both stable and clear in appearance and are effective as lubricants.

According to this invention, there is provided a lubricant composition comprising an organosilicon component (A) and a polyoxyethylene alkylphenol Component (B), characterised in that Component (A) is (i) a polydimethyl siloxane of the average general formula

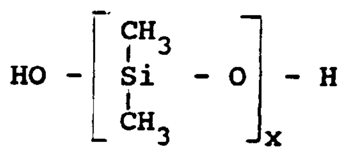

wherein x has a value of from 3 to 51, and/or (ii) a silanol of the general formula $R'_2CH_3SiOH$ wherein each R' denotes a methyl or phenyl group, at least one R' being phenyl and in that Component (B) is dissolved in Component (A) and has the general formula R-Ph-$[OCH_2CH_2]_nOH$, wherein R represents an alkyl group having from 6 to 12 carbon atoms, Ph represents a phenylene group and n has a value of from 8 to 10.

Also included in the scope of the invention is the use of said compositions as spermicidal lubricants, especially for condoms.

Polydimethyl siloxanes which are useful as Component (A) (i) in the compositions of the present invention are substantially linear materials. They have silanol groups at the terminal positions of the polymer molecule. A particularly useful material is α,w-dimethylsilanol end-blocked polydimethylsiloxane. Such polymers and methods for preparing them are well known in the art of silicone technology. They may be obtained for example by the hydrolysis of dimethyldichlorosilane and, if necessary, polymerisation of the low molecular weight hydrolysis product thereof. The silanol-terminated polydimethylsiloxanes are widely employed in the silicone rubber art and differ from the so-called "silicone fluids" by the presence of terminal dimethylsilanol ($-Si(CH_3)_2OH$) groups rather than trimethylsilyl ($-Si(CH_3)_3$). Silanols (A) (ii) and methods for their manufacture are also well known in the silicone technology.

The preferred polydimethyl siloxanes (A) (i) are those which have from 4 to 30 silicon atoms in the chain. It is especially preferred that they have from 5 to 15 silicon atoms present in the siloxane chain. A suitable polydimethylsiloxane has the average formula $HO-[Si(CH_3)_2-O]_9-H$.

The polyoxyethylene alkylphenols (B), for use in the composition according to the invention, are preferably those wherein n has a value of about 9. R may be e.g. a hexyl, heptyl or octyl group, preferably a nonyl group, for example 3,5,5-trimethylhexyl.

Lubricant compositions according to the invention are stable and clear in appearance. It is preferred that the compositions consist only of Components (A) and (B), although other components may be present. If other components are present they preferably include only small amounts, e.g. traces of polydimethylsiloxane which have terminal silanol groups, but which have a larger number of silicon atoms in the siloxane chain or of volatile silicones, e.g. polydimethyl cyclosiloxanes and hexamethyl disiloxane. It is, however, preferred for most spermicidal and virucidal applications that the polydimethylsiloxane (A) (i) is as pure as possible. This can be achieved e.g. by well known purification methods such as distillation or thin film evaporation in order to remove any volatile materials which may be present. The preferred silicone portion (A) of the composition comprises a polydimethylsiloxane (A) (i) or a mixture of two or more polydimethylsiloxanes (A) (i). Especially preferred are those compositions in which Component (A) consist only of the most preferred polydimethylsiloxanes (A) (i), i.e. those having from 5 to 15 silicon atoms.

The amount of Component (B) used in the lubricant compositions may vary from 0.1 to about 70% by weight

of the combined weight of (A) and (B), more preferably from 2 to 60%. For most applications however, no more than about 50% percent by weight of (B) is used, based on the combined weight of (A) and (B). When the silanol (A) (ii) is used clear solutions result when (A) and (B) are present in any ratio; the preferred proportions of (A) (ii) and (B) are the same as those mentioned above and also apply when Component (A) (i) is used. When Component (A) (i) is used in combination with small amounts of Component (B), e.g. less than 20% by weight based on the total weight of (A) and (B), clear solutions are obtained. If higher levels of Component (B) are to be used, such as 20% or more, best results in clarity are achieved when the preferred polydimethylsiloxanes (A) (i) are used. A more preferred range of proportions of (A) and (B) is from 2 to 25% by weight of the preferred material (B) by weight of (A) and (B) combined, most preferably from 3 to 10% by weight.

The lubricant compositions according to the invention are readily prepared by mixing Components (A) and (B) in the desired proportions. It is preferred to choose the components such that the final viscosity of the composition at 25°C would be less than 500 mm²/s, as this will ease the processibility of the composition. For most applications the preferred viscosity would be in the range of from 40 to 150 mm²/s, most preferably 50 to 100 mm²/s.

The compositions according to the invention are useful in applications requiring a liquid composition having spermicidal or virucidal properties. They are particularly suitable for use as lubricants for condoms and in other contraceptive applications. Alternative applications may include the treatment of surgical gloves or different pieces of surgical apparatus for specialised operations and treatments.

The invention accordingly also comprises condoms which use as a lubricant those compositions which are according to the invention.

The following Examples illustrate the invention. All parts and percentages are given by weight, unless otherwise indicated, and Me denotes a methyl group.

Example 1

8g of polyethyleneoxy nonylphenol having 9 ethyleneoxy groups were stirred into 92g of a polydimethylsiloxane having the average formula

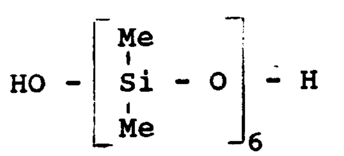

The resulting product was a clear liquid having a viscosity of 40 mm²/s at 25°C.

Example 2

64g of polyethyleneoxy nonylphenol having 9 ethyleneoxy groups were stirred into 36g of the polydimethylsiloxane employed in Example 1. The resulting product was a clear liquid having a viscosity of 120 mm²/s at 25°C.

Example 3

10g of polyethyleneoxy nonylphenol having 9 ethyleneoxy groups were stirred into 90g of diphenylmethylsilanol. The resulting product was a clear liquid having a viscosity of 180 mm²/s at 25°C.

Example 4

20 parts of polyethyleneoxy nonylphenol having 9 ethyleneoxy groups were stirred into 80 parts of a polydimethylsiloxane having the average formula

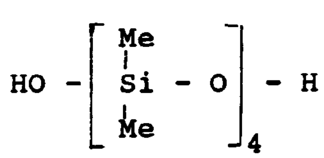

The resulting product was a clear liquid.

Example 5

60 parts of polyethyleneoxy nonylphenol having 9 ethyleneoxy groups were stirred into 40 parts of a polydimethylsiloxane having the average formula

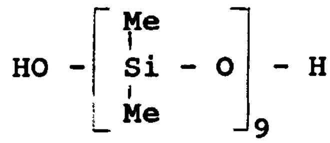

The resulting product was a clear liquid.

Example 6

4 parts of polyethyleneoxy nonylphenol having 9 ethyleneoxy groups were stirred into 96 parts of a polydimethylsiloxane having the average formula

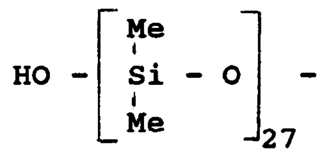

The resulting product was a clear liquid.

Example 6

30 parts of polyethyleneoxy nonylphenol having 9 ethyleneoxy groups were stirred into 70 parts of a mixture of the polydimethylsiloxanes used in Examples 5 and 6 respectively, in a proportion of 8/2. The resulting product was a clear liquid having a viscosity of 60 $mm^2/s$ at 24°C.

Example 7

24 parts of polyethyleneoxy nonylphenol having 9 ethyleneoxy groups were stirred into 76 parts of a mixture of the polydimethylsiloxanes used in Examples 5 and 6 respectively, in a proportion of 6/4. The resulting product was a clear liquid having a viscosity of 58 $mm^2/s$ at 24°C.

Example 8

15 parts of polyethyleneoxy nonylphenol having 9 ethyleneoxy groups were stirred into 85 parts of a mixture of the polydimethylsiloxanes used in Examples 5 and 6 respectively, in a proportion of 4/6. The resulting product was a clear liquid having a viscosity of 54 $mm^2/s$ at 24°C.

Example 9

8 parts of polyethyleneoxy nonylphenol having 9 ethyleneoxy groups were stirred into 92 parts of a mixture of the polydimethylsiloxanes used in Examples 5 and 6 respectively, in a proportion of 8/2. The resulting product was a clear liquid having a viscosity of 51 $mm^2/s$ at 24°C.

## Claims

1. A lubricant composition comprising an organosilicon Component (A) and a polyoxyethylene alkylphenol Component (B), characterised in that Component (A) is (i) a polydimethylsiloxane of the average general formula

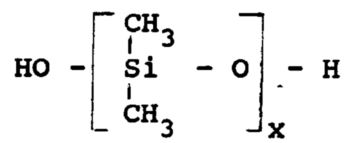

wherein x has a value of from 3 to 51, and/or (ii) a silanol of the general formula $R'_2CH_3SiOH$ wherein each R' denotes a methyl or phenyl group, at least one R' being phenyl, and in that Component (B) is dissolved in Component (A) and has the general formula R-Ph-$[OCH_2CH_2]_nOH$, wherein R represents an alkyl group having from 6 to 12 carbon atoms, Ph represents a phenylene group and n has a value of from 8 to 10.

2. A composition according to Claim 1 characterised in that polydimethylsiloxane (A) (i) has from 4 to 30 silicon atoms in the chain.

3. A composition according to Claim 1 or 2 characterised in that polydimethylsiloxane (A) (i) has from 5 to 15 silicon atoms present in the siloxane chain.

4. A composition according to anyone of the preceding claims characterised in that in polyoxyethylene alkylphenol (B) n has the average value of 9 and R represents a nonyl group.

5. A composition according to anyone of the preceding claims characterised in that the percentage by weight of Component (B) based on the combined weight of (A) and (B) is from 0.1 to 70%.

6. A composition according to anyone of the preceding claims characterised in that the percentage by weight of Component (B) based on the combined weight of (A) and (B) is from 2 to 25%.

7. A composition according to anyone of the preceding claims characterised in that the final viscosity of the composition at 25°C is less than $500mm^2/s$.

8. A composition according to anyone of the preceding claims characterised in that the final viscosity of the composition at 25°C is in the range of from 50 to $100mm^2/s$.

9. A method of making a lubricant compositions according to any one of the preceding claims which comprises mixing (A) (i) a polydimethyl siloxane of the average general formula

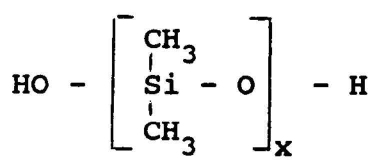

wherein x has a value of from 3 to 51, and/or (ii) a silanol of the general formula $R'_2CH_3SiOH$ wherein each R' denotes a methyl or phenyl group, at least one R' being phenyl, with (B) a polyoxyethylene alkylphenol of the general formula R-Ph-$[OCH_2CH_2]_nOH$ wherein R represents an alkyl group having from 6 to 12 carbon atoms, Ph represents a phenylene group and n has a value of from 8 to 10 in the desired proportions.

10. The use of a composition according to any one of Claims 1 to 9 as a spermicidal lubricant.

11. The use of a composition according to any one of Claims 1 to 9 as a spermicidal lubricant for condoms.

12. The use of a composition according to any one of Claims 1 to 9 as a virucidal lubricant.

13. A condom comprising a lubricant composition characterised in that said composition comprises (A) (i) a polydimethylsiloxane of the average general formula

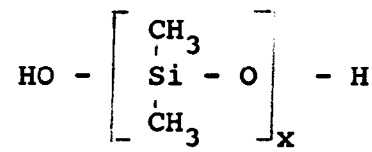

wherein x has a value of from 3 to 51, and/or (ii) a silanol of the general formula $R'_2CH_3SiOH$ wherein each R' denotes a methyl or phenyl group, at least one R' being phenyl, having dissolved therein (B) a polyoxyethylene alkylphenol of the general formula $R\text{-}Ph\text{-}[OCH_2CH_2]_nOH$, wherein R represents an alkyl group having from 6 to 12 carbon atoms, Ph represents a phenylene group and n has a value of from 8 to 10.

## Patentansprüche

1. Gleitmittelzusammensetzung umfassend eine Organosiliciumkomponente (A) und eine polyoxyethylenalkylphenolkomponente (B), dadurch gekennzeichnet, daß Komponente (A) (i) ein Polydimethylsiloxan der durchschnittlichen allgemeinen Formel

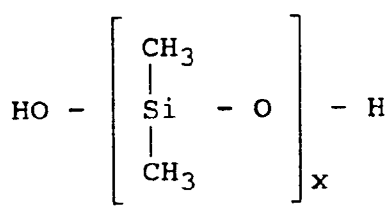

ist, worin x einen Wert von 3 bis 51 hat, und/oder (ii) ein Silanol der allgemeinen Formel
$$R'_2CH_3SiOH$$
ist, worin jeder Rest R' eine Methyl- oder Phenylgruppe bezeichnet, wobei mindestens ein Rest R' ein Phenylrest ist, und daß die Komponente (B) in Komponente (A) gelöst wird und die allgemeine Formel
$$R\text{-}Ph\text{-}[OCH_2CH_2]_nOH$$
hat, worin R eine Alkylgruppe mit 6 bis 12 Kohlenstoffatomen bedeutet, Ph eine Phenylengruppe bedeutet und n einen Wert von 8 bis 10 hat.

2. Zusammensetzung nach Anspruch 1, dadurch gekennzeichnet, daß das Polydimethylsiloxan (A)(i) 4 bis 30 Siliciumatome in der Kette aufweist.

3. Zusammensetzung nach Anspruch 1 oder 2, dadurch gekennzeichnet, daß das Polydimethylsiloxan (A)(i) 5 bis 15 Siliciumatome in der Siloxankette aufweist.

4. Zusammensetzung nach einem der vorhergehenden Ansprüche, dadurch gekennzeichnet, daß in dem Polyoxyethylenalkylphenol (B) n einen Durchschnittswert von 9 hat und R eine Nonylgruppe bedeutet.

5. Zusammensetzung nach einem der vorhergehenden Ansprüche, dadurch gekennzeichnet, daß der Gewichtsprozentanteil der Komponente (B), bezogen auf das Gesamtgewicht von (A) und (B), 0,1 bis 70% ist.

6. Zusammensetzung nach einem der vorhergehenden Ansprüche, dadurch gekennzeichnet, daß der Gewichtsprozentanteil von Komponente (B), bezogen auf das Gesamtgewicht von (A) und (B), 2 bis 25% ist.

7. Zusammensetzung nach einem der vorhergehenden Ansprüche, dadurch gekennzeichnet, daß die endgültige Viskosität der Zusammensetzung bei 25°C geringer als 500 $mm^2$/Sekunde ist.

8. Zusammensetzung nach einem der vorhergehenden Ansprüche, dadurch gekennzeichnet, daß die endgültige Viskosität der Zusammensetzung bei 25°C im Bereich von 50 bis 100 $mm^2$/Sekunde liegt.

9. Verfahren zur Herstellung einer Gleitmittelzusammensetzung nach einem der vorhergehenden Ansprüche, umfassend, daß man (A)(i) ein Polydimethylsiloxan der durchschnittlichen allgemeinen Formel

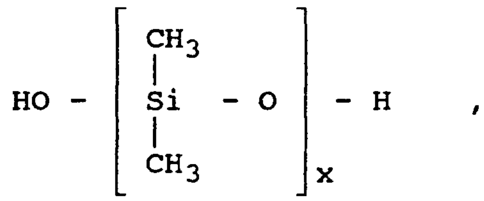

worin x einen Wert von 3 bis 51 hat, und/oder (ii) ein Silanol der allgemeinen Formel

$R'_2CH_3SiOH$ ,

worin jeder Rest R' eine Methyl- oder Phenylgruppe bezeichnet, wobei mindestens ein Rest R' ein Phenylrest ist, mit (B) einem Polyoxyethylenalkylphenol der allgemeinen Formel

$R\text{-}Ph\text{-}[OCH_2CH_2]_nOH$ ,

worin R eine Alkylgruppe mit 6 bis 12 Kohlenstoffatomen bedeutet, Ph eine Phenylengruppe bedeutet und n einen Wert von 8 bis 10 hat, in den gewünschten Anteilen vermischt.

**10.** Verwendung einer Zusammensetzung nach einem der Ansprüche 1 bis 9 als spermizides Gleitmittel.

**11.** Verwendung einer Zusammensetzung nach einem der Ansprüche 1 bis 9 als spermizides Gleitmittel für Kondome.

**12.** Verwendung einer Zusammensetzung nach einem der Ansprüche 1 bis 9 als viruzides Gleitmittel.

**13.** Kondom umfassend eine Gleitmittelzusammensetzung, dadurch gekennzeichnet, daß die Zusammensetzung (A) (i) ein Polydimethylsiloxan der durchschnittlichen allgemeinen Formel

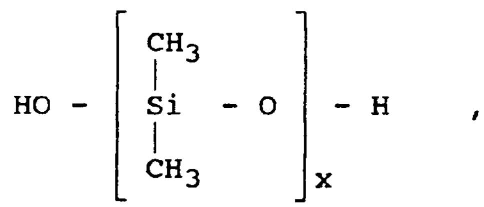

worin x einen Wert von 3 bis 51 hat, und/oder (ii) ein Silanol der allgemeinen Formel

$R'_2CH_3SiOH$ ,

worin jeder Rest R' eine Methyl- oder Phenylgruppe bezeichnet, wobei mindestens ein Rest R' ein Phenylrest ist, die ein Polyoxyethylenalkylphenol der allgemeinen Formel

$R\text{-}Ph\text{-}[OCH_2CH_2]_nOH$ ,

worin R eine Alkylgruppe mit 6 bis 12 Kohlenstoffatomen bedeutet, Ph eine Phenylengruppe bedeutet und n einen Wert von 8 bis 10 hat, darin gelöst enthält, umfaßt.

## Revendications

**1.** Composition de lubrifiant comprenant un constituant organosilicié (A) et un constituant alkylphénol polyéthoxylé (B), caractérisée en ce que le constituant (A) est (i) un polydiméthylsiloxane de la formule générale moyenne :

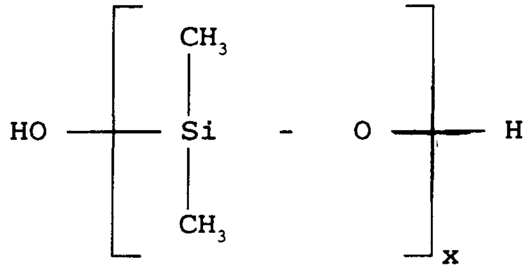

où la valeur de x est de 3 à 51, et/ou (ii) un silanol de formule générale $R'_2CH_3SiOH$ où chaque R' est un groupe méthyle ou phényle, au moins un R' étant un groupe phényle, et en ce que le constituant (B) est dissous dans le constituant (A) et a la formule générale

$$R - Ph - [OCH_2CH_2]_nOH$$

où R représente un groupe alkyle ayant 6 à 12 atomes de carbone, Ph représente un groupe phénylène et la valeur de n est de 8 à 10.

2. Composition selon la revendication 1, caractérisée en ce que le polydiméthylsiloxane (A) (i) a de 4 à 30 atomes de silicium dans la chaîne.

3. Composition selon la revendication 1 ou 2, caractérisée en ce que le polydiméthylsiloxane (A) (i) a de 5 à 15 atomes de silicium présents dans la chaîne siloxane.

4. Composition selon l'une quelconque des revendications précédentes, caractérisée en ce que, dans l'alkylphénol polyéthoxylé (B), la valeur de n est 9 et R représente un groupe nonyle.

5. Composition selon l'une quelconque des revendications précédentes, caractérisée en ce que le pourcentage en poids du constituant (B) va de 0,1 à 70%, par rapport au poids total de (A) et (B).

6. Composition selon l'une quelconque des revendications précédentes, caractérisée en ce que le pourcentage en poids du constituant (B) par rapport au poids total de (A) et (B) va de 2 à 25%.

7. Composition selon l'une quelconque des revendications précédentes, caractérisée en ce que la viscosité finale de la composition à 25°C est inférieure **à** 500 $mm^2/s$.

8. Composition selon l'une quelconque des revendications précédentes, caractérisée en ce que la viscosité finale de la composition à 25°C est dans la gamme allant de 50 à 100 $mm^2/s$.

9. Procédé de préparation de compositions lubrifiantes selon l'une quelconque des revendications précédentes, qui consiste à mélanger (A) (i) un polydiméthylsiloxane de la formule générale moyenne :

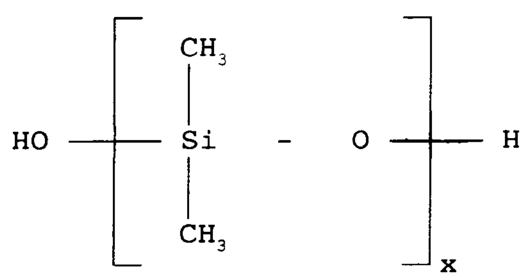

où la valeur de x est de 3 à 51, et/ou (ii) un silanol de formule générale $R'_2CH_3SiOH$ où chaque R' est un groupe méthyle ou phényle, au moins un R' étant un groupe phényle, avec (B) un alkylphénol polyéthoxylé de la formule générale

$$R - Ph - [OCH_2CH_2]_nOH$$

où R représente un groupe alkyle ayant 6 à 12 atomes de carbone, Ph représente un groupe phénylène et la valeur de n est de 8 à 10.

**10.** Utilisation d'une composition selon l'une quelconque des revendications 1 à 9 comme lubrifiant spermicide.

**11.** Utilisation d'une composition selon l'une quelconque des revendications 1 à 9 comme lubrifiant spermicide pour préservatifs.

**12.** Utilisation d'une composition selon l'une quelconque des revendications 1 à 9 comme lubrifiant virucide.

**13.** Préservatif comprenant une composition lubrifiante, caractérisé en ce que ladite composition comprend un polydiméthylsiloxane de la formule générale moyenne :

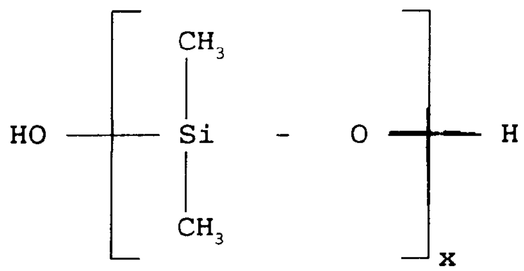

où la valeur de x est de 3 à 51, et/ou (ii) un silanol de formule générale $R'_2CH_3SiOH$ où chaque R' est un groupe méthyle ou phényle, au moins un R' étant un groupe phényle, dans lequel est dissous (B) un alkylphénol polyéthoxylé de la formule générale

$$R - Ph - [OCH_2CH_2]_nOH$$

où R représente un groupe alkyle ayant 6 à 12 atomes de carbone, Ph représente un groupe phénylène et la valeur de n est de 8 à 10.